# EUROPÄISCHE PATENTSCHRIFT

(11) **EP 2 136 127 B1**
(45) Veröffentlichungstag und Bekanntmachung des Hinweises auf die Patenterteilung: **13.10.2010**
(21) Anmeldenummer: 08011295.6
(22) Anmeldetag: 20.06.2008
(51) Int. Cl.: F21S 8/00, F21V 21/40, A61B 19/00

(54) **Operationsleuchte mit beleuchteten Handgriffen**
Operating light with illuminated handles
Lampe chirurgicale dotée de poignées éclairées

(43) Veröffentlichungstag der Anmeldung: 23.12.2009
(73) Patentinhaber: TRUMPF Medizin Systeme GmbH + Co. KG, 82178 Puchheim (DE)
(72) Erfinder: Fritze, Dirk, 82275 Emmering (DE); Tahbazian, Kamran, 82194 Gröbenzell (DE); Marka, Rudolf, 85737 Ismaning (DE)
(74) Vertreter: Prüfer & Partner GbR European Patent Attorneys

(56) Entgegenhaltungen:
- EP-A- 1 568 934
- WO-A-2007/014769
- US-A1- 2003 014 834
- US-A1- 2003 210 559

## Beschreibung

Die Erfindung betrifft eine Operationsleuchte, die beleuchtete Handgriffe aufweist.

Operationsleuchten werden an einer Raumdecke eines Operationssaals, einer Wand, oder als fahrbare Ausführungen an einem fahrbaren Ständer mit Hilfe eines so genannten Tragsystem befestigt. Das Tragsystem besteht üblicherweise aus einem ersten Ausleger, der um eine vertikale Achse schwenkbar ist und einem zweiten Ausleger, der horizontal schwenkbar und höhenverstellbar ist. Mit dem Tragsystem besteht die Möglichkeit, die Operationsleuchte an einer Stelle im Raum zu positionieren, von der aus es günstig ist, die Operationsstelle auszuleuchten.

Üblicherweise sind Operationsleuchten mit einem Griff, der im sterilen Bereich der Operationsleuchte angeordnet ist, und mit dem der sterile Operateur die Operationsleuchte positionieren kann, ausgerüstet.

Zur Positionierung der Operationsleuchte vor der Operation oder während der Operation durch unsteriles Operationspersonal sind die Operationsleuchten meistens mit weiteren Griffen am äußeren Umfang des Leuchtenkörpers ausgestattet.

In den letzten Jahren vermehren sich die operativen Eingriffe in minimalinvasiver Operationstechnik. Diese Operationen werden ohne große Operationsöffnungen nur mit kleinen Öffnungen, durch die die Optik und die Instrumente eingeführt werden durchgeführt. Das Bild der minimalinvasiven Operationsstelle wird durch ein Kamerasystem auf Monitore, die auch an dem Tragsystem der Operationsleuchte angeordnet sein können, angezeigt. Bei dieser Operationstechnik ist zu Beginn der Operation helles Licht der Operationsleuchte erforderlich, um die Öffnung für die Operation zu schaffen. Während der minimalinvasiven Operation wird die Operationsleuchte ausgeschaltet und das Raumlicht im Operationssaal abgedunkelt, um für den Operateur die Sichtbedingungen auf die Monitore, auf die das Bild der Operationsstelle von dem Endoskop übertragen wird, zu verbessern. Dabei ergibt sich das Problem, dass die Position der Operationsleuchte nicht mehr einfach erkennbar ist, was zu Problemen beim Wiedereinschalten der Operationsleuchte führen kann, was zum Schließen der Wunden und zum Beenden der Operation erforderlich ist.

Die Patentanmeldung WO 2007/014769 A1 offenbart eine gattungs bildende Operationsleuchte mit einem Zusatzleuchtmittel zur Steigerung der Leistungsfähigkeit des Operationsteams, das unabhängig von dem Hauptleuchtmittel, das ein Operationsfeld ausleuchtet, schaltbar ist. Das Zusatzleuchtmittel ist über dem gesamten Umfang am oberen Rand des Handgriffs angeordnet und mit einem Diffusor versehen.

In der Patentanmeldung EP 1 568 934 ist eine Operationsleuchte gezeigt, die in dem zentralen Handgriff eine zuschaltbare Lichtquelle enthält, die ein zusätzliches Lichtfeld auf der Operationsstelle zum Ausleuchten von tiefen Wunden mit geringem Durchmesser erzeugt.

Es ist Aufgabe der Erfindung, dieses Problem zu lösen. Insbesondere ist es Aufgabe der Erfindung, eine Operationsleuchte mit Elementen bereitzustellen, die einfach und kostengünstig beleuchtbar sind, und die eine leichte Erkennung der Position und der Orientierung der Operationsleuchte ermöglichen.

Die Aufgabe wird mit den Kennzeichmenden Merkmalen des Anspruchs 1 gelöst. Weiterbildungen der Erfindung sind Gegenstand der Unteransprüche.

Durch die Integration der Beleuchtungselemente in die Handgriffe ist die einfache, kostengünstige Möglichkeit gegeben, die Operationsleuchte ohne zusätzliche Verkleidungs- oder Halterungsbauteile mit Elementen zu versehen, aus denen Licht austritt. Die Lichtaustrittsfläche ist transluzent, d.h. lichtdurchlässig, ohne die Lichtquellen, wie bei einem transparenten Material sichtbar werden zu lassen.

In einer weiteren Ausgestaltung der Erfindung weist die transluzente Fläche eine strukturierte Oberfläche, d.h. eine erhabene Musterung der Oberfläche, wie sie z.B. bei sandgestrahlten, geriffelten oder kreuzgeriffelten Oberflächen erzeugt wird, auf. Dabei wird eine Oberfläche mit kugelförmigen Vertiefungen, dreiecksförmigen länglichen Erhöhungen, bzw. pyramidenförmigen Erhebungen erzeugt. Die Vertiefungen bzw. Erhöhungen bewegen sich dabei im Hundertstelmillimeter-Bereich. Somit tritt das Licht diffus aus den zweiten Lichtaustrittsflächen und man kann die Herkunft des Lichts, also das Leuchtmittel, nicht erkennen.

Die Orientierung von Normalen, die an verschiedenen Orten auf der zweiten Lichtaustrittsfläche senkrecht stehen, in mindestens zwei verschiedenen Richtungen, d.h. die Ausrichtung von zwei verschiedenen Lichtaustrittsflächen unter einem bestimmten Raumwinkel, oder eine Krümmung von nur einer Lichtaustrittsfläche, ermöglicht das deutlichere Erkennen des Lichts aus verschiedenen Blickrichtungen.

Der Handgriff ist aus Gründen einer guten Erreichbarkeit an dem äußeren Umfang des Leuchtenkörpers angebracht, kann aber auch in einer alternativen Ausführungsform am Tragsystem angeordnet sein.

In einer weiteren Ausgestaltung der Erfindung wird die Farbe des Lichts, das von dem Leuchtmittel ausgestrahlt wird, durch die Lichtaustrittsfläche nicht verändert. D.h. Licht, das durch ein weißes Leuchtmittel ausgestrahlt wird, bleibt auch nach dem Durchtritt durch die Lichtaustrittsfläche weiß.

Hingegen wird in einer alternativen Ausführungsform der Erfindung die Farbe des ausgestrahlten Lichts durch die Lichtaustrittsfläche verändert. Von dem Leuchtmittel abgegebenes weißes Licht wird durch die Lichtaustrittsfläche beispielsweise in blaues ausgestrahltes Licht verändert.

Wird das Leuchtmittel so angeschlossen, dass eine Stromversorgung der Operationsleuchte angezeigt wird, so ist an der Operationsleuchte erkennbar, ob die Operationsleuchte durch einen externen Hauptschalter stromlos geschaltet wurde, oder ob sie sich in einem Standby-Modus befindet und direkt an der Operationsleuchte eingeschaltet werden kann.

In dem Fall, in dem die Operationsleuchte von verschiedenen Stromversorgungen, beispielsweise einerseits von einer normalen Stromversorgung aus einem konventionellen Versorgungsnetz, oder andererseits einer speziellen Stromversorgung mit einem batteriegepufferten Notstromaggregat, das innerhalb von kürzester Zeit einen Ausfall der normalen Stromversorgung kompensiert, versorgt wird, ist es wichtig die Art der Stromversorgung zu erkennen. Dabei ist das Leuchtmittel so angeschlossen, dass es bei der Versorgung durch die spezielle Stromversorgung leuchtet und diese somit angezeigt wird. Das Anzeigen kann beispielsweise durch ein Leuchten des Leuchtmittels oder durch Blinken des Leuchtmittels in einem bestimmten Rhythmus erfolgen.

Alternativ zum Leuchten oder Blinken des Leuchtmittels wird in einer weiteren Ausführungsform die Farbe des ausgestrahlten Lichts bei der Versorgung durch die spezielle Stromversorgung geändert. So wird beispielsweise durch die externe Umschaltung der Versorgung auf die spezielle Stromversorgung ein Farbfilter zwischen das Leuchtmittel und die Lichtaustrittsfläche eingebracht.

Der Betrieb mit der Versorgung mit der speziellen Stromversorgung wird in einer Weiterbildung durch mindestens ein zweites, spezielles Leuchtmittel angezeigt, das der Handgriff aufweist. Das spezielle Leuchtmittel strahlt dann eine andere Farbe aus, als das Leuchtmittel, wodurch der Betrieb mit der speziellen Stromversorgung angezeigt wird.

In einer anderen Ausführung ist ein drittes Leuchtmittel so angeschlossen, dass es einen bestimmten Betriebszustand, beispielsweise den Betrieb bei einer normgerechten Lichtstärkeverteilung im Leuchtfeld, anzeigt.

In einer weiteren Ausführungsform hat ein Teil der zweiten Lichtaustrittsfläche einen refraktorartigen Aufbau. Dadurch wird das Licht von dem vierten Leuchtmittel innerhalb des Griffs so fokussiert, dass bei ausgeschalteten Leuchtmitteln in der ersten Lichtaustrittsfläche des Leuchtenkörpers das Operationsfeld für minimalinvasive Operationen beleuchtet wird.

Dafür ist in einer alternativen Ausbildung ein weiteres viertes Leuchtmittel vorhanden, das Licht ausstrahlt, das durch den Refraktor fokussiert und ausgestrahlt wird.

Für spezielle Anwendung, beispielsweise für die Diagnostik, hat das Licht des vierten Leuchtmittels die Charakteristik, dass es nur Schwingungen in einem sehr engen Wellenlängenbereich aufweist. Beispielsweise wird in Kombination mit dem Fluorophor Fluorescein, das in das zu untersuchende Gewebe injiziert wird, ein Leuchtmittel verwendet, das eine Wellenlänge von 450 ± 5 Nanometer aufweist, wodurch das Fluorophor zum Strahlen angeregt wird. So kann über einen Filter, der nur das von dem Fluorphor ausgestrahlte Licht passieren lässt, eine Gewebeerkennung durchgeführt werden.

Die Erfindung wird anhand eines Ausführungsbeispiels unter Bezugnahme auf die beigefügten Zeichnungen erläutert.
- Fig. 1: ist eine perspektivische Ansicht eines Ausführungsbei- spiels der erfindungsgemäßen Operationsleuchte.
- Fig. 2: zeigt einen Teil einer geschnittenen Seitenansicht ei- nes Leuchtenkörpers mit einem Handgriff.
- Fig. 3: zeigt eine perspektivische Ansicht eines Innenteils eines Handgriffs mit Leuchtmittel.
- Fig. 4: zeigt eine perspektivische Ansicht eines Außenteils eines Handgriffs.

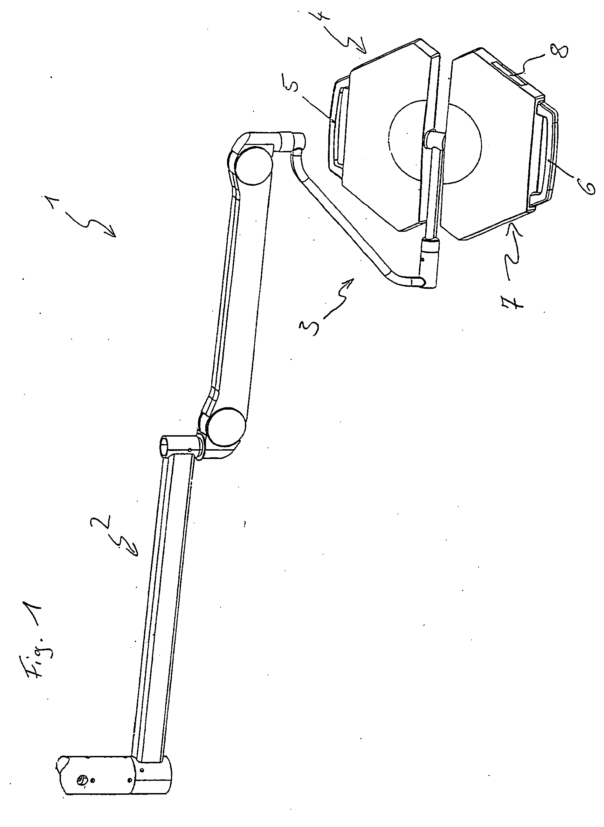

Fig. 1 zeigt eine perspektivische Ansicht eines Ausführungsbeispiels der erfindungsgemäßen Operationsleuchte 1. Die Operationsleuchte 1 umfasst ein Tragsystem 2, eine Aufhängevorrichtung 3 und einen Leuchtenkörper 4. Das Tragsystem 2 wird an einer Raumdecke, einer Wand oder einem fahrbaren Ständer befestigt. Durch das Tragsystem 2 und die Aufhängevorrichtung 3 ist der Leuchtenkörper 4 innerhalb des Aktionsradius in jeder beliebigen räumlichen Stellung und Orientierung positionierbar.

Zum unsterilen Positionieren des Leuchtenkörpers 4 sind an den beiden Hälften des Leuchtenkörpers 4 je ein Handgriff 5, 6 angebracht. Die beiden Hälften des Leuchtenkörpers 4 sind drehfest miteinander verbunden, so dass sich beim Verschwenken der einen Hälfte die andere Hälfte mit bewegt, um die Lichtaustrittsflächen immer in einer Ebene zu halten.

Innerhalb des Leuchtenkörpers 4 ist eine später beschriebene Steuerungsvorrichtung 7 angeordnet, um lange Kabelverbindungen zu vermeiden. Die Steuerungsvorrichtung muss jedoch nicht zwingend in dem Leuchtenkörper 4 angeordnet sein, sondern kann auch, um den Leuchtenköper möglichst klein und strömungsgünstig zu gestalten, in einem separaten Gehäuse, das am Leuchtenkörper 4 oder an der Aufhängevorrichtung 3 angeordnet ist, untergebracht sein. Alternativ besteht auch die Möglichkeit, dass sich die Steuerungsvorrichtung 7 in einer nicht gezeigten externen Bedieneinheit befindet, die sich in einer Medizinischen Versorgungseinheit oder in/an einer Wand befindet.

An der Außenseite des Leuchtenkörpers 4 ist eine Bedienvorrichtung 8 mit Schalt- und Einstellelementen angeordnet. Die Bedienvorrichtung 8 kann sich aber auch in einem separaten Gehäuse befinden, das sich beispielsweise am Leuchtenkörper 4, an der Aufhängevorrichtung 3 oder in einer Medizinischen Versorgungseinheit oder in/an einer Wand befindet.

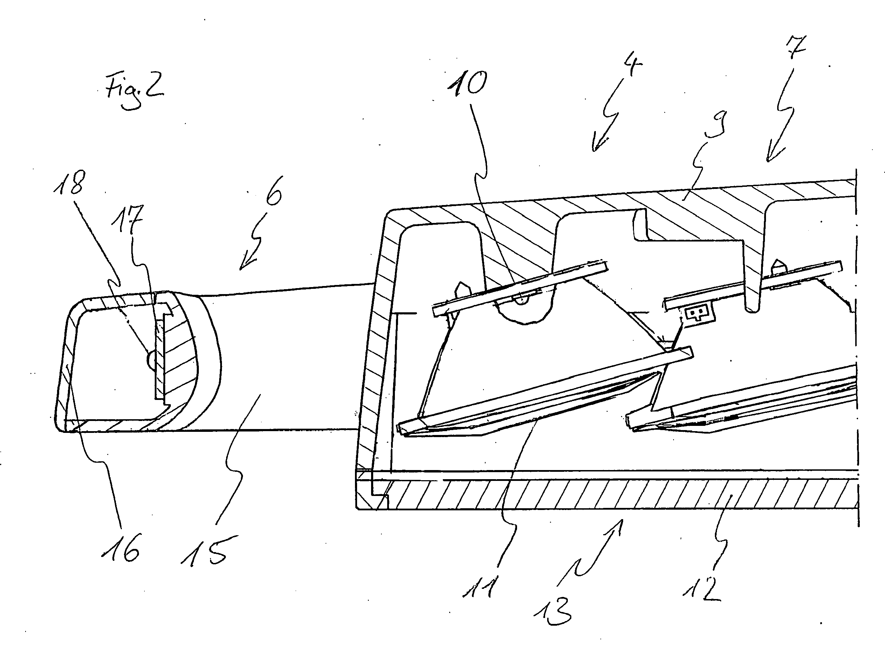

Fig. 2 zeigt einen Teil einer geschnittenen Seitenansicht eines Leuchtenkörpers 4 mit einem Handgriff 6. In einem Gehäuse 9 des Leuchtenkörpers 4, das aus Aluminiumdruckguss besteht, sind Leuchtmittel 10 an schrägen Befestigungsflächen befestigt. Die Leuchtmittel 10 strahlen über Refraktoren 11 gebündelte Lichtstrahlen durch eine untere transparente Abdeckscheibe 12 aus Kunststoff, die die erste Lichtaustrittsfläche 13 bildet, aus.

Die Abdeckscheibe 12 ist an dem Gehäuse 9 befestigt. Die Lichtstrahlen sind auf ein Operationsfeld, das sich in einem bestimmten Abstand befindet, gerichtet und bilden dort ein Leuchtfeld als einen Bereich, der durch die Lichtstrahlen beleuchtet wird.

Der Handgriff 6 besteht aus zwei Teilen, einem Innenteil 15 und einem Außenteil 16. Das Innenteil 15 ist seitlich durch vier hier nicht gezeigte Schrauben mit dem Gehäuse 9 verbunden. Das Außenteil 16 ist über eine Clipverbindung mit dem Innenteil 15 verbunden.

An dem Innenteil 15 ist mit Hilfe eines Klebebands ein Leiterplattenstreifen 17 befestigt. Auf dem Leiterplattenstreifen 17 sind zehn Leuchtmittel 18, hier LEDs, befestigt und elektrisch verbunden. Der Leiterplattenstreifen wird über nicht gezeigte Kabel versorgt.

In dem Gehäuse 9 des Leuchtenkörpers 4 befindet sich die Steuerungsvorrichtung 7. Die Steuerungsvorrichtung 7 weist Mittel zum Dimmen und Ein- und Ausschalten der Leuchtmittel 10, wie z.B. Stromregler, Mittel zum Übertragen von Schalt- und Einstellinformationen der Schalt- und Einstellelemente der Bedienvorrichtung 8, einen Speicherbereich zum Abspeichern von Betriebsparametern und eine CPU, die aus den Schalt- und Einstellinformationen, an Hand der abgespeicherten Betriebsparameter, die erforderlichen Einstellungen für die Mittel zum Dimmen und Ein- und Ausschalten der Leuchtmittel 10 berechnet oder bestimmt, auf.

Die Steuerungsvorrichtung 7 ist mit einer nicht gezeigten Stromversorgungsvorrichtung, den Leuchtmitteln 18 und den Leuchtmitteln 10, sowie der Bedienvorrichtung 8 mit einem Element zum Ein-/Ausschalten verbunden.

An der Bedienvorrichtung 8 (Fig. 1) ist
- ein Element zum Ein-/Ausschalten,
- ein Element zur Abstandseinstellung und
- ein Element zur Helligkeitseinstellung
vorgesehen.

In einer alternativen Ausführungsform ist die Bedienvorrichtung beleuchtet. Hierbei besteht optional die Möglichkeit, die gesamte Bedienvorrichtung, die angezeigten Symbole und die Betätigungselemente alternativ oder gemeinsam zu beleuchten.

Das Element zum Ein-/Ausschalten schaltet die Operationsleuchte 1 von einem Standby-Modus, in dem die Leuchtmittel 10 nicht leuchten, in einen Betriebsmodus und zurück. Im Betriebsmodus werden die Leuchtmittel 10 entsprechend der Einstellungen der Einstellelemente an der Bedienvorrichtung 8 betrieben. Zum vollständigen Ausschalten durch Abschalten der Stromversorgung ist ein nicht gezeigter externer Hauptschalter vorgesehen. Bei einem Abschalten der Stromversorgung erlöschen die Leuchtmittel 18. Ansonsten leuchten die Leuchtmittel 18 sowohl im Standby-Modus als auch im Betriebsmodus der Operationsleuchte 1.

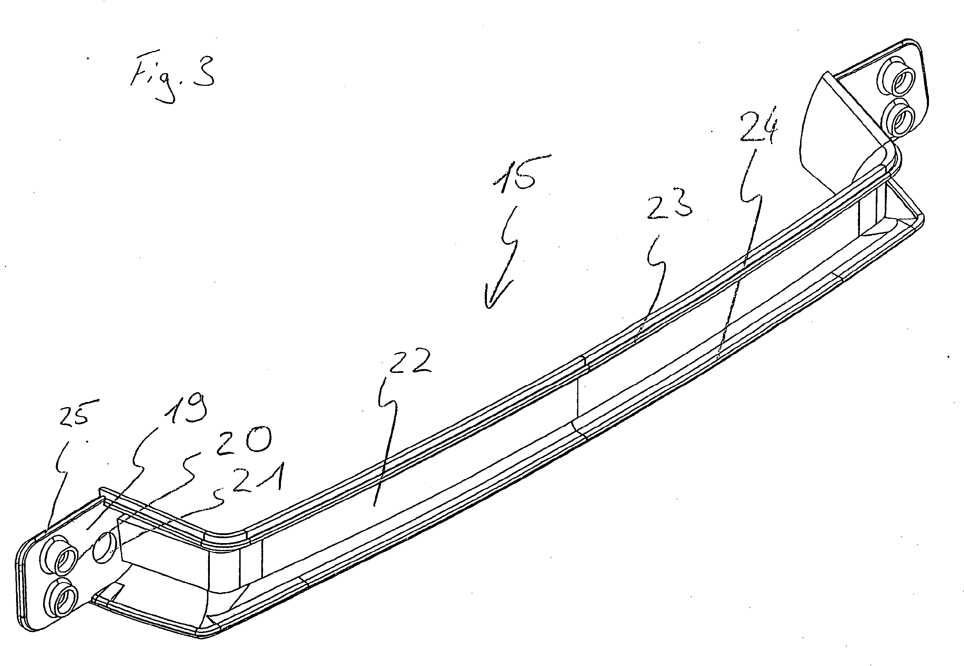

Fig. 3 zeigt eine perspektivische Ansicht des Innenteils 15 des Handgriffs 5, 6 mit Leuchtmittel 18. Es ist ergonomisch ausgeformt und an seinen beiden Enden jeweils mit einer Anschraublasche 19 versehen. In diesen Anschraublaschen 19 sind jeweils zwei Dome 20 zur Befestigung des Innenteils, mit Hilfe von jeweils zwei Schrauben, an dem Gehäuse 9 vorgesehen. An den Anschraublaschen sind ebenfalls jeweils eine Bohrung 21 zum Durchführen von nicht gezeigten Verbindungskabeln zwischen der Steuerungsvorrichtung 7 und dem o.a. Leiterplattenstreifen 17 in das Gehäuse 9 vorgesehen.

Auf der Innenseite des Innenteils 15 ist in seiner Längsrichtung eine Materialverdickung vorgesehen, die eine Befestigungsfläche 22 für den Leiterplattenstreifen 17 bildet. An den Außenseiten eines oberen und unteren Rands des Innenteils 15 sind jeweils umlaufend von der Befestigungsfläche 22 bis zur gegenüberliegenden Befestigungsfläche ein Teil einer Clipverbindung 23, 24 angeordnet. Auch an den beiden Anschraublaschen 19 ist umlaufend ein Teil einer Clipverbindung 25 angeordnet. Das Innenteil 15 ist aus einem transluzentem Kunststoffmaterial im Spritzgussverfahren hergestellt. Durch die Herstellung aus dem transluzentem Kunststoffmaterial, stellen, mit Ausnahme der Anschraublaschen 19 und der Befestigungsfläche 22, alle von außen sichtbaren Flächen zweite Lichtaustrittsflächen dar.

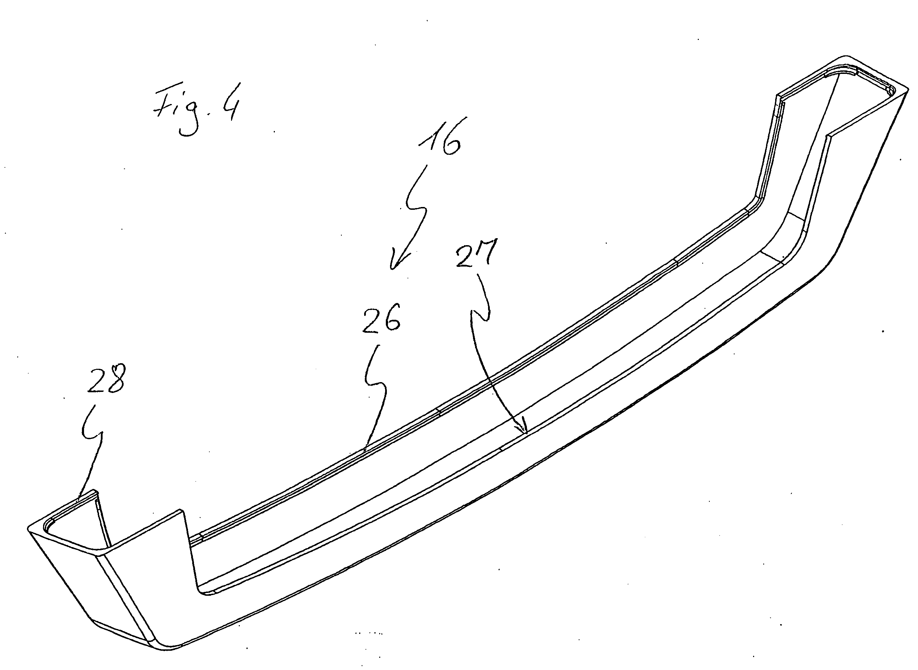

Fig. 4 zeigt eine perspektivische Ansicht des Außenteils 16 des Handgriffs 5, 6. Das Außenteil ist passgenau zu der Form des Innenteils 15 und ebenfalls ergonomisch ausgeformt. Insbesondere sind die umlaufenden Kanten, an denen Gegenstücke 26, 27, 28 zu den Clipsverbindungen 23, 24, 25 angeordnet sind, mit dem zugehörigen oberen und unteren Rand des Innenteils 15, sowie den Befestigungsflächen 22 zu einer Bildung eines möglichst engen, gleichmäßigen Spalts abgestimmt, um hygienische Anforderungen zu erfüllen. Das Außenteil 16 ist aus dem gleichen transluzentem Kunststoffmaterial wie das Innenteil 15 im Spritzgussverfahren hergestellt.
Somit stellen auch hier alle von außen sichtbaren Flächen die zweiten Lichtaustrittsflächen dar.

## Patentansprüche

1. Operationsleuchte mit einem Tragsystem (2), einem Leuchtenkörper (4) mit einer ersten Lichtaustrittsfläche (13), und mindestens einem Handgriff (5, 6) mit mindestens einem Leuchtmittel (18), wobei der Handgriff (5, 6) zumindest teilweise eine zweite Lichtaustrittsfläche aufweist und die zweite Lichtaustrittsfläche transluzent ist, **dadurch gekennzeichnet, dass** annähernd der gesamte Handgriff (5, 6) die zweite Lichtaustrittsfläche aufweist.

2. Operationsleuchte gemäß Anspruch 1, **dadurch gekennzeichnet, dass** die zweite Lichtaustrittsfläche eine strukturierte Oberfläche aufweist und das Licht des Leuchtmittels (18) diffus austritt.

3. Operationsleuchte gemäß einem der Ansprüche 1 oder 2, **dadurch gekennzeichnet, dass** die zweite Lichtaustrittsfläche so gestaltet ist, dass Normale, die senkrecht an verschiedenen Orten auf der zweiten Lichtaustrittsfläche stehen, in mindestens zwei Richtungen orientiert sind.

4. Operationsleuchte gemäß einem der vorangehenden Ansprüche, **dadurch gekennzeichnet, dass** der Handgriff (5, 6) am äußeren Umfang des Gehäuses (9) des Leuchtenkörpers (4) befestigt ist.

5. Operationsleuchte gemäß einem der vorangehenden Ansprüche, **dadurch gekennzeichnet, dass** die zweite Lichtaustrittsfläche die Farbe des vom Leuchtmittel (18) ausgestrahlten Lichts nicht verändert.

6. Operationsleuchte gemäß einem der vorangehenden Ansprüche, **dadurch gekennzeichnet, dass** die zweite Lichtaustrittsfläche die Farbe des vom Leuchtmittel (18) ausgestrahlten Lichts verändert.

7. Operationsleuchte gemäß einem der vorangehenden Ansprüche, **dadurch gekennzeichnet, dass** das Leuchtmittel (18) so angeschlossen ist, dass eine Stromversorgung der Operationsleuchte angezeigt wird.

8. Operationsleuchte gemäß einem der vorangehenden Ansprüche, **dadurch gekennzeichnet, dass** die Operationsleuchte alternativ mit einer normalen und mit einer speziellen Stromversorgung versorgt wird und ein Leuchtmittel so angeschlossen ist, dass ein Betrieb mit der speziellen Stromversorgung angezeigt wird.

9. Operationsleuchte gemäß Anspruch 8, **dadurch gekennzeichnet, dass** das ausgestrahlte Licht bei dem Betrieb mit der speziellen Stromversorgung eine andere Farbe aufweist, als bei einem Betrieb mit der normalen Stromversorgung.

10. Operationsleuchte gemäß einem der Ansprüche 1 bis 7, **dadurch gekennzeichnet, dass** die Operationsleuchte alternativ mit einer normalen und mit einer speziellen Stromversorgung versorgt wird und der Handgriff (5, 6) mindestens ein zweites, spezielles Leuchtmittel aufweist, das einen Betrieb mit der speziellen Stromversorgung anzeigt.

11. Operationsleuchte gemäß Anspruch 10, **dadurch gekennzeichnet, dass** das Licht, das von dem speziellen Leuchtmittel ausgestrahlt wird, eine andere Farbe, als das von dem Leuchtmittel (18) ausgestrahlte Licht, aufweist.

12. Operationsleuchte gemäß einem der vorangehenden Ansprüche, **dadurch gekennzeichnet, dass** ein drittes Leuchtmittel so angeschlossen ist, dass ein bestimmter Betriebszustand der Operationsleuchte (1), vorzugsweise eine normgerechte Lichtverteilung im Leuchtfeld, angezeigt wird.

13. Operationsleuchte gemäß einem der vorangehenden Ansprüche, **dadurch gekennzeichnet, dass** ein Teil der zweiten Lichtaustrittsfläche einen refraktorartigen Aufbau hat.

14. Operationsleuchte gemäß Anspruch 13, **dadurch gekennzeichnet, dass** das Licht durch den Teil mit dem refraktorartigen Aufbau von mindestens einem vierten Leuchtmittel ausgestrahlt wird.

15. Operationsleuchte gemäß Anspruch 14, **dadurch gekennzeichnet, dass** das Licht des vierten Leuchtmittels eine schmalbandige Wellenlänge, vorzugsweise 450 ± 5 Nanometer, aufweist.

## Claims

1. Operating lamp having a carrying system (2), a lamp body (4) with a first light emerging face (13), and at least one handle (5, 6) having at least one illuminant (18), wherein the handle (5, 6) at least partially comprises a second light emerging face and the second light emerging face is translucent, **characterized in that** approximately the entire handle (5, 6) comprises the second light emerging face.

2. Operating lamp according to claim 1, **characterized in that** the second light emerging face comprises a structured surface and the light of the illuminant (18) diffusely emerges.

3. Operating lamp according to any of claims 1 or 2, **characterized in that** the second light emerging face is designed such that normals being perpendicular on the second light emerging face at different locations are oriented in at least two directions.

4. Operating lamp according to any of the preceding claims, **characterized in that** the handle (5, 6) is fixed at the outer circumference of the housing (9) of the lamp body (4).

5. Operating lamp according to any of the preceding claims, **characterized in that** second light emerging face does not modify the colour of the light emitted by the illuminant (18).

6. Operating lamp according to any of the preceding claims, **characterized in that** the second light emerging face modifies the colour of the light emitted by the illuminant (18).

7. Operating lamp according to any of the preceding claims, **characterized in that** the illuminant is connected in such a way that an electricity supply of the operating lamp is indicated.

8. Operating lamp according to any of the preceding claims, **characterized in that** the operating lamp is alternatively supplied by a normal and by a special electricity supply and an illuminant is connected in such a way that an operation with the special electricity supply is indicated.

9. Operating lamp according to claim 8, **characterized in that** the emitted light has a colour which is different during operation with the special electricity supply than during operation with the normal electricity supply.

10. Operating lamp according to any of claims 1 to 7, **characterized in that** the operating lamp is alternatively supplied by a normal electricity supply and by a special electricity supply and the handle (5, 6) comprises at least one second specific illuminant indicating an operation with the special electricity supply.

11. Operating lamp according to claim 10, **characterized in that** the light emitted by the specific illuminant has a colour which is different from the colour of the light emitted by the illuminant (18).

12. Operating lamp according to any of the preceding claims, **characterized in that** a third illuminant is connected in such a way that a particular operating condition of the operating lamp (1), preferably a standardized light distribution in the illuminated field, is indicated.

13. Operating lamp according to any of the preceding claims, **characterized in that** a portion of the second light emerging face has a refractor-like structure.

14. Operating lamp according to claim 13, **characterized in that** the light through the portion with the refractor-like structure is emitted by at least a fourth illuminant.

15. Operating lamp according to claim 14, **characterized in that** the light of the fourth illuminant has a narrow-banded wavelength, preferably 450 ± 5 nanometre.

## Revendications

1. Lampe chirurgicale avec un système support (2), un corps de lampe (4) avec une première surface de sortie de lumière (13), et au moins une poignée (5, 6) avec au moins un élément luminescent (18), la poignée (5, 6) présentant au moins partiellement une deuxième surface de sortie de lumière et la deuxième surface de sortie de lumière étant translucide, **caractérisée en ce qu'**à peu près l'ensemble de la poignée (5, 6) présente la deuxième surface de sortie de lumière.

2. Lampe chirurgicale selon la revendication 1, **caractérisée en ce que** la deuxième surface de sortie de lumière présente une surface structurée et la lumière de l'élément luminescent (18) sort de façon diffuse.

3. Lampe chirurgicale selon l'une des revendications 1 ou 2, **caractérisée en ce que** la deuxième surface de sortie de lumière est configurée de manière que des normales, tracées perpendiculairement en différents emplacements sur la deuxième surface de sortie de lumière, soient orientées en au moins deux directions.

4. Lampe chirurgicale selon l'une des revendications précédentes, **caractérisée en ce que** la poignée (5, 6) est fixée sur la périphérie extérieure du boîtier (9) du corps de lampe (4).

5. Lampe chirurgicale selon l'une des revendications précédentes, **caractérisée en ce que** la deuxième surface de sortie de lumière ne modifie pas la couleur de la lumière irradiée par l'élément luminescent (18).

6. Lampe chirurgicale selon l'une des revendications précédentes, **caractérisée en ce que** la deuxième surface de sortie de lumière modifie la couleur de la lumière irradiée par l'élément luminescent (18).

7. Lampe chirurgicale selon l'une des revendications précédentes, **caractérisée en ce que** l'élément luminescent (18) est raccordé de manière qu'une alimentation en courant électrique des lampes chirurgicales soit affichée.

8. Lampe chirurgicale selon l'une des revendications précédentes, **caractérisée en ce que** la lampe chirurgicale est alternativement alimentée avec une alimentation en courant électrique normale et avec une alimentation en courant électrique spéciale, et un élément luminescent est raccordé de manière qu'un fonctionnement avec l'alimentation en courant électrique spéciale soit affichée.

9. Lampe chirurgicale selon la revendication 8, **caractérisée en ce que**, lors du fonctionnement avec l'alimentation en courant électrique spéciale, la lumière irradiée présente une couleur autre que lors du fonctionnement avec l'alimentation en courant électrique normale.

10. Lampe chirurgicale selon l'une des revendications 1 à 7, **caractérisée en ce que** la lampe chirurgicale est alternativement alimentée avec une alimentation en courant électrique normale et avec une alimentation en courant électrique spéciale, et la poignée (5, 6) présente au moins un deuxième élément luminescent, spécial, affichant un fonctionnement avec l'alimentation en courant électrique spéciale.

11. Lampe chirurgicale selon la revendication 10, **caractérisée en ce que** la lumière irradiée par l'élément luminescent spécial présente une couleur autre que la lumière irradiée par l'élément luminescent (18).

12. Lampe chirurgicale selon l'une des revendications précédentes, **caractérisée en ce qu'**un troisième élément luminescent est raccordé de manière qu'un état de fonctionnement déterminé de la lampe chirurgicale (1), de préférence une distribution de lumière conforme aux normes dans le champ d'éclairage, est indiqué.

13. Lampe chirurgicale selon l'une des revendications précédentes, **caractérisée en ce qu'**une partie de la deuxième surface de sortie de lumière présente une structure de type réfracteur.

14. Lampe chirurgicale selon la revendication 13, **caractérisée en ce que** la lumière est irradiée à travers la partie ayant la structure de type réfracteur d'au moins un quatrième élément luminescent.

15. Lampe chirurgicale selon la revendication 14, **caractérisée en ce que** la lumière du quatrième élément luminescent présente une longueur d'onde à bande étroite, de préférence de 450 ± 5 nanomètres.
